(19) Europäisches Patentamt
European Patent Office
Office européen des brevets

(11) EP 2 005 943 A2

(12) # EUROPEAN PATENT APPLICATION
published in accordance with Art. 153(4) EPC

(43) Date of publication:
**24.12.2008 Bulletin 2008/52**

(21) Application number: **07741273.2**

(22) Date of filing: **09.04.2007**

(51) Int Cl.:
***A61K 8/73*** (2006.01) ***A61K 8/06*** (2006.01)
***A61K 8/19*** (2006.01) ***A61K 8/34*** (2006.01)
***A61Q 19/10*** (2006.01) ***C11D 1/74*** (2006.01)

(86) International application number:
**PCT/JP2007/057837**

(87) International publication number:
**WO 2007/116999 (18.10.2007 Gazette 2007/42)**

(84) Designated Contracting States:
**DE ES FR GB**

(30) Priority: **12.04.2006 JP 2006109675**
**18.10.2006 JP 2006284017**

(71) Applicant: **KAO CORPORATION**
**Tokyo 103-8210 (JP)**

(72) Inventors:
- **NISHIOKA, Toru**
  **Wakayama-shi, Wakayama 640-8580 (JP)**
- **IHARA, Takeshi**
  **Wakayama-shi, Wakayama 640-8580 (JP)**

(74) Representative: **HOFFMANN EITLE**
**Patent- und Rechtsanwälte**
**Arabellastrasse 4**
**81925 München (DE)**

(54) **W/O/W TYPE EMULSIFIED COMPOSITION**

(57) The present invention relates to a W/O/W emulsion composition that is excellent in emulsification stability, and exhibits a good spreadability over skins and imparts a good wet feel upon use without stickiness and oiliness; a detergent composition containing a W/O/W emulsion which is excellent in touch feel after washing without deterioration in washing performance; and processes for producing these compositions. According to the present invention, there are provided a W/O/W emulsion composition including a polysaccharide derivative containing in a side chain thereof, a group represented by the formula (1):

$$-(OX)_n-E^1-R \qquad (1);$$

a detergent composition containing the W/O/W emulsion; and process for producing these composition.

EP 2 005 943 A2

## Description

Field of the Invention

The present invention relates to W/O/W emulsion compositions, detergent compositions containing a W/O/W emulsion, and processes for producing these compositions.

Background of the Invention

The W/O/W emulsion compositions are also referred to as composite emulsions or multiple emulsions, and have been used as important materials in various industrial applications such as cosmetics, food and drugs or medicines. The W/O/W emulsion compositions have such a structure in which an oil phase is dispersed in water as an outer phase, and further water as an inner phase is dispersed in the oil phase, and can exhibit a good feeling upon use which is never obtained by O/W type emulsions or W/O type emulsions. In addition, the W/O/W emulsion compositions containing an effective substance enclosed within W/O droplets thereof can also be used in various applications including cosmetics, food and drugs or medicines.
However, the W/O/W emulsion compositions are extremely deteriorated in emulsification stability and therefore tend to suffer from fusion between the inner water phase and the outer water phase as well as unification of the oil phase. As a result, it is known that the W/O/W emulsion compositions finally cause separation between the oil phase and the water phases.
In particular, in detergents containing the W/O/W emulsion, the emulsification stability of the W/O/W emulsion tends to be damaged by a surfactant contained in the detergents, so that the detergents are hardly produced with a good stability.
Conventionally, various attempts have been made in order to improve the emulsification stability of the W/O/W emulsion compositions. For example, Patent Document 1 discloses a method for producing a W/O/W emulsion composition by dispersing a W/O type emulsion containing an emulsifier having a HLB value of 7 or less and an electrolyte in an outer water phase containing an alkyl-modified carboxyvinyl polymer and a carboxyvinyl polymer. Also, Patent Document 2 discloses a method for producing a W/O/W emulsion composition composed of an inner water phase containing a hydrophilic emulsifier having a HLB value of 7 or more which is made of an ester of a fatty acid and a polyhydric alcohol, an oil phase containing a lipophilic emulsifier made of an ester of a fatty acid having 16 to 22 carbon atoms which contains 50% or more of an unsaturated fatty acid, or a condensed oxy fatty acid, and a polyhydric alcohol having 4 or more carbon atoms, and an outer water phase containing a hydrophilic emulsifier having a HLB value of 5 or more. However, these methods for producing the W/O/W emulsion compositions are such a method of retaining a good emulsification stability by forming a high-viscous creamy composition. Therefore, the conventional W/O/W emulsion compositions are still unsatisfactory in properties when used in applications requiring a low viscosity.
Further, from the reasons as described above, it has been very difficult to stably blend the W/O/W emulsion in detergent compositions.
Patent Document 3 discloses a novel polysaccharide derivative exhibiting an excellent water solubility and having a specific thickening effect of exhibiting a higher viscosity than its viscosity at room temperature in a higher temperature range than room temperature as well as an effect of highly stabilizing a hydrophobic substance present in water. However, the Patent Document 3 fails to disclose a specific method of allowing W/O/W emulsions containing the polysaccharide derivative to retain a good emulsification stability.
Furthermore, Patent Document 4 discloses an oil-in-water (W/O) type emulsion capable of being blended in a detergent using the above novel polysaccharide derivative and exhibiting an excellent applying feel to skins, as well as a process for producing the emulsion. However, the emulsion tends to suffer from problems such as creaming when used in low-viscous products owing to a large size of emulsified particles contained therein.
Patent Document 1: JP 2002-275029A
Patent Document 2: JP 2001-25360A
Patent Document 3: WO 00/73351
Patent Document 4: JP 2003-226612A

Summary of the Invention

The present invention relates to the following aspects (1), (2), (3) and (4).

(1) A W/O/W emulsion composition including a polysaccharide derivative containing in a side chain thereof, a group represented by the following general formula (1):

$$-(OX)_n-E^1-R \qquad (1)$$

wherein X is a linear or branched divalent saturated hydrocarbon group having 1 to 6 carbon atoms; n is a number of 5 to 300; a plurality of X groups in number of n may be the same or different; $E^1$ is an ether bond (-O-) or an ester bond (-OCO- or -COO-); and R is a linear or branched hydrocarbon group having 4 to 30 carbon atoms which may be substituted with hydroxyl group.
(2) A detergent composition including a W/O/W emulsion composition containing the polysaccharide derivative as described in the above aspect (1).
(3) A process for producing a W/O/W emulsion composition, including the steps of:

mixing a composition (X) containing (i) a polysaccharide derivative containing in a side chain thereof, a group represented by the following general formula (1):

$$-(OX)_n-E^1-R \qquad (1)$$

wherein X is a linear or branched divalent saturated hydrocarbon group having 1 to 6 carbon atoms; n is a number of 5 to 300; a plurality of X groups in number of n may be the same or different; $E^1$ is an ether bond (-O-) or an ester bond (-OCO- or -COO-); and R is a linear or branched hydrocarbon group having 4 to 30 carbon atoms which may be substituted with hydroxyl group, and (ii) a water-soluble polyol, with a W/O emulsion (Y) previously produced from (iii) water, (iv) a hydrophobic compound and (v) an emulsifier having a HLB value of 7 or less, to obtain a mixed composition (Z); and
diluting the resultant mixed composition (Z) with water.

(4) A process for producing a detergent composition containing a W/O/W emulsion, including the steps of producing the mixed composition as described above, and diluting the resultant mixed composition with (vi) an aqueous detergent solution.

Detailed Description of the Invention

The present invention has been made to solve the above problems encountered in the prior art, and relates to a W/O/W emulsion composition having an excellent emulsification stability and exhibiting a good spreadability and a good wet feel upon application onto skins without stickiness and oiliness, a detergent composition containing a W/O/W emulsion which is excellent in not only emulsification stability without deterioration in washing performance, but also touch feel after washing, and processes for producing these compositions.
The present inventors have found that the above problems can be solved by the W/O/W emulsion composition and a detergent composition containing a W/O/W emulsion which contain a novel polysaccharide derivative having an excellent water solubility, a specific thickening effect and an effect of stabilizing a hydrophobic substance.
The present invention relates to the W/O/W emulsion composition including the polysaccharide derivative containing in a side chain thereof, a group represented by the above general formula (1).
In the present invention, the polysaccharide derivative means a polysaccharide and a derivative thereof.
Specific examples of the polysaccharide include polysaccharides such as cellulose, guar gum, starch, pullulan, dextran, fructan, mannan, inulin, agar, carrageenan, chitin, chitosan, pectin, alginic acid, and hyaluronic acid; and derivatives thereof obtained by introducing a substituent group such as methyl, ethyl, hydroxyethyl and hydroxypropyl into these polysaccharides. These substituent groups may be introduced into a constitutional monosaccharide residual group of the polysaccharide, either singly or in combination of any two or more thereof.
Specific examples of the derivatives of these polysaccharides include hydroxyethyl cellulose, hydroxyethylethyl cellulose, hydroxyethyl guar gum, hydroxyethyl starch, methyl cellulose, methyl guar gum, methyl starch, ethyl cellulose, ethyl guar gum, ethyl starch, hydroxypropyl cellulose, hydroxypropyl guar gum, hydroxypropyl starch, hydroxyethylmethyl cellulose, hydroxyethylmethyl guar gum, hydroxyethylmethyl starch, hydroxypropylmethyl cellulose, hydroxypropylmethyl guar gum and hydroxypropylmethyl starch. Among these polysaccharides or derivatives thereof, preferred are cellulose, starch, hydroxyethyl cellulose, methyl cellulose, ethyl cellulose and hydroxypropyl cellulose, and more preferred is hydroxyethyl cellulose.
These polysaccharides or derivatives thereof preferably have a weight-average molecular weight of from 10,000 to 10,000,000, more preferably from 10,000 to 2,000,000 and still more preferably from 30,000 to 1,500,000.

(i) Polysaccharide Derivative

In the polysaccharide derivative (i) used in the present invention which contains in a side chain thereof a group represented by the general formula (1), X in the general formula (1) is preferably an alkylene group having 2 to 4 carbon atoms and more preferably an ethylene group; n is preferably a number of 5 to 200 and more preferably 8 to 120; and R is preferably an alkyl group having 4 to 30 carbon atoms and more preferably 6 to 25 carbon atoms.
Examples of the polysaccharide derivative include substituted polysaccharides obtained by substituting a part or whole of hydrogen atoms of hydroxyl groups of a polysaccharide or a derivative thereof with at least one group selected from the group consisting of the following groups (A) to (D):

(A) a group represented by the following general formula (2):

$$-E^2-(OX)_m-E^1-R \qquad (2)$$

wherein $E^2$ is a linear or branched divalent saturated hydrocarbon group having 1 to 6 carbon atoms which may be substituted with hydroxyl group or oxo group; m is a number of 8 to 300; X, $E^1$ and R are the same as defined above; and a plurality of X groups in number of m may be the same or different;
(B) a sulfoalkyl group having 1 to 5 carbon atoms which may be substituted with hydroxyl group, or a salt thereof;
(C) a carboxyalkyl group having 2 to 6 carbon atoms which may be substituted with hydroxyl group, or a salt thereof; and
(D) a group represented by the following general formula (3):

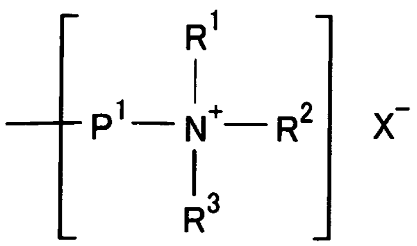

(3)

wherein $P^1$ is a linear or branched divalent saturated hydrocarbon group having 1 to 6 carbon atoms which may be substituted with hydroxyl group; $R^1$, $R^2$ and $R^3$ may be the same or different, and are respectively a linear or branched alkyl group having 1 to 3 carbon atoms which may be substituted with hydroxyl group; and; and $X^-$ is a hydroxyl ion, a halogen ion or an organic acid ion.
In addition, the substituted polysaccharides may be those obtained by further substituting a hydrogen atom of the hydroxyl group contained in the respective groups (A) to (D) with the group (A), (B), (C) or (D).
In the substituted polysaccharides obtained by substituting a part or whole of hydrogen atoms of a polysaccharide or a derivative thereof with the group (A) and optionally with the group (B), (C) and/or (D) in which celluloses are used, for example, as the polysaccharide or the derivative thereof, the repeating unit contained therein is represented, for example, by the following general formula (4):
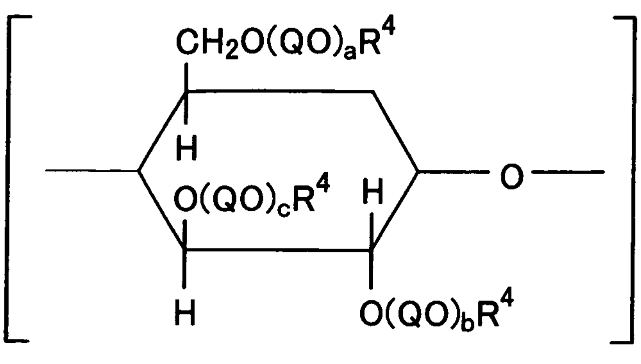

(4)

wherein $R^4$ is a group selected from the group consisting of a hydrogen atom, methyl, ethyl, hydroxyethyl, hydroxypropyl, the substituent group (A) represented by the above general formula (2) containing a polyoxyalkylene group, the above sulfoalkyl group (B), the above carboxyalkyl group (C), and the above cationic substituent group (D) represented by the general formula (3); Q is an alkylene group having 2 to 4 carbon atoms; a, b and c are each a number of 0 to 10; and the QO groups, the $R^4$ groups, and suffixes a, b and c may be the same or different within each repeating unit or between the respective repeating units. Further, the respective hydroxyl groups contained in the above substituent groups (A) to (D) may be further substituted with the other group selected from the substituent groups (A) to (D), with the proviso that $R^4$ contains at least the substituent group (A).

Substituent Group (A)

In the general formula (2) representing the substituent group (A) containing a polyoxyalkylene group, $E^2$ is preferably a linear or branched divalent saturated hydrocarbon group having 2 or 3 carbon atoms which may be substituted with hydroxyl group or oxo group. Specific examples of the preferred $E^2$ include ethylene, propylene, trimethylene, 2-hydroxytrimethylene, 1-hydroxymethylethylene, 1-oxoethylene, 1-oxotrimethylene and 1-methyl-2-oxoethylene. X in the general formula (2) is preferably a linear or branched divalent saturated hydrocarbon group having 2 or 3 carbon atoms. Specific examples of the preferred X include ethylene, propylene and trimethylene. The polymerization degree of (-OX-) represented by m is preferably from 8 to 120 and more preferably from 10 to 60 from the viewpoints of a good thickening effect and a good emulsification stability. A plurality of the X groups in number of m may be the same or different. Here, m means an average molar number of addition of the (-OX-) groups.
$E^1$ is an ether bond or an oxycarbonyl group, and is preferably an ether bond. R in the general formula (2) is preferably a linear or branched alkyl group having 5 to 20 carbon atoms and especially 6 to 20 carbon atoms which may be substituted with hydroxyl group, and more preferably an unsubstituted alkyl group and still more preferably an unsubstituted linear alkyl group from the viewpoint of a good stability. Specific examples of the preferred R include octyl, decyl, dodecyl, tetradecyl, hexadecyl, octadecyl and isostearyl.
The degree of substitution with the substituent group (A) in the substituted polysaccharides used in the present invention is preferably from 0.0001 to 1.0, more preferably from 0.0005 to 0.5 and still more preferably from 0.001 to 0.1 per a constitutional monosaccharide residual group thereof.

Substituent Group (B)

Examples of the substituent group (B), i.e., the sulfoalkyl group having 1 to 5 carbon atoms which may be substituted with hydroxyl group, or the salt thereof, include 2-sulfoethyl, 3-sulfopropyl, 3-sulfo-2-hydroxypropyl and 2-sulfo-1-(hydroxymethyl)ethyl. Among these groups, from the viewpoints of a good stability and a good productivity, preferred is 3-sulfo-2-hydroxypropyl. These substituent groups (B) may be partially or wholly in the form of a salt with an element belonging to Group 1 or 2 of the Periodic Table such as Na, K, Ca and Mg, an organic cation derived from amines or ammonium, etc. The degree of substitution with the substituent group (B) in the substituted polysaccharides is preferably from 0 to 1.0, more preferably from 0 to 0.8 and still more preferably from 0 to 0.5 per a constitutional monosaccharide residual group thereof.

Substituent Group (C)

Examples of the substituent group (C), i.e., the carboxyalkyl group having 2 to 6 carbon atoms which may be substituted with hydroxyl group, or the salt thereof, include carboxymethyl, carboxylethyl, carboxypropyl, carboxylbutyl and carboxypentyl. Among these groups, from the viewpoints of a good stability and a good productivity, preferred is carboxymethyl. These substituent groups (C) may be partially or wholly in the form of a salt with an element belonging to Group 1 or 2 of the Periodic Table such as Na, K, Ca and Mg, an organic cation derived from amines or ammonium, etc. The degree of substitution with the substituent group (C) in the substituted polysaccharides is preferably from 0 to 1.0, more preferably from 0 to 0.8 and still more preferably from 0 to 0.5 per a constitutional monosaccharide residual group thereof.

Substituent Group (D)

$P^1$ contained in the cationic substituent group (D) represented by the following general formula (3):
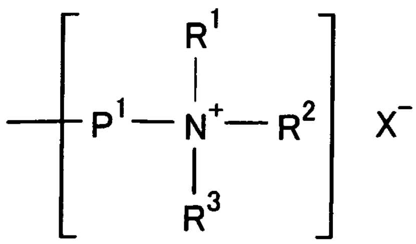

(3)

wherein $P^1$, $R^1$, $R^2$, $R^3$ and $X^-$ are the same as defined above, is preferably a linear or branched divalent saturated hydrocarbon group having 2 or 3 carbon atoms which may be substituted with hydroxyl group. Specific examples of the preferred $P^1$ include ethylene, propylene, trimethylene, 2-hydroxytrimethylene and 1-hydroxymethylethylene. Examples of $R^1$, $R^2$ and $R^3$ in the cationic substituent group (D) include methyl, ethyl, propyl and 2-hydroxyethyl. Among these groups, preferred are methyl and ethyl. Examples of the halogen ion represented by $X^-$ in the cationic substituent group (D) include a chlorine ion, a bromine ion and an iodine ion. Examples of the organic acid ion represented by $X^-$ in the cationic substituent group (D) include $CH_3COO^-$, $CH_3CH_2COO^-$ and $CH_3(CH_2)_2COO^-$. Among these groups as $X^-$, preferred are a hydroxyl ion, a chorine ion and a bromine ion.

The degree of substitution with the cationic substituent group (D) in the substituted polysaccharides is preferably from 0 to 0.5 and more preferably from 0 to 0.3 per a constitutional monosaccharide residual group thereof.

The substitution of the polysaccharide or salt thereof with the respective substituent groups (A) to (D), i.e., polyoxyalkylenation, sulfoalkylation, carboxyalkylation or cationization of the polysaccharide or salt thereof, may be carried out by the method as described in WO 00/73351.

(ii) Water-Soluble Polyol Optionally Containing Water

The water-soluble polyol used in the present invention is in the form of a polyhydric alcohol containing two or more hydroxyl groups in a molecule thereof. Specific examples of the water-soluble polyol include alkylene glycols such as ethylene glycol, propylene glycol, 1,3-butylene glycol and 1,4-butylene glycol; polyalkylene glycols such as dipropylene glycol; sugar alcohols such as glucose, maltose, maltitose, sucrose, fructose, xylitol, sorbitol, maltotriose and threitol; glycerol; polyglycerol; erythritol; and starch-decomposed reduced alcohols. These water-soluble polyols may be used alone or in combination of any two or more thereof.

In the detergent of the present invention, among them, from the viewpoint of a good stability of the W/O/W emulsion composition in the finally obtained detergent system, preferred are 1,3-butylene glycol and glycerol, and more preferred is 1,3-butylene glycol.

In addition, the W/O/W emulsion composition may be prepared by using water containing no water-soluble polyol. From the viewpoint of a good stability of the obtained emulsion, the mass ratio of the water-soluble polyol to water (water-soluble polyol/water) is preferably from 0/100 to 50/50.

(iii) Water

Water used in the present invention serves for forming the inner water phase, and may contain an effective substance. Examples of the effective substance used in the inner water phase include those substances that are ordinarily blended in toiletry products for enhancing a function and an added value thereof, such as plant extracts, amino acids, anti-inflammatory agents and antioxidants.

(Plant Extracts)

Examples of the plant extracts include those extracts obtained by ordinary methods from plants such as Angelica keiskei, adzuki bean, avocado, hydrangea, Gynostemma pentaphyllum, altheca, arnica, almond, aloe, apricot, stinging nettle, iris, fennel, turmeric, rosa multiflora, Scutellaria baicalensis, Phellodendron amurense, Coptis japonica, barley, gumbo, Saint-John's-wort, dead nettle, Ononis spinosa, Nasturtium officinale, persimmon, puerariae root, Valeriana fauriei, birch, cattail, chamomile, chamomilla, oats, licorice, raspberry, kiwi, cucumber, Armeniacae semen, kukui nut, Cape jasmine, Sasa albo-marginata, walnut, cinnamon, mulberry, Gunjo, gentian, cranesbill, burdock, sesame, wheat, rice, Camellia sasanqua, saffron, hawthorn, Japanese pepper tree, mushroom, Rehmannia glutinosa, Chicon, beefsteak plant, Japanese linden, Filipendula multijuga, peony, ginger, Japanese iris, white birch, Lonicera japonica, field horsetail, Stevia rebaudiana Bertoni, western ivy, western hawthorn, black elder, Juniperus communis, Achillea millefolium, peppermint, sage, common mallow, Cinidium officinale, Japanese green gentian, soybean, Zizyphi fructus, thyme, tea,

clove, dried orange peel, evening primrose, camellia, Centella asiatica, English walnut, Angelica acutiloba, Calendula officinalis, Amygdalus persica, Picea jezoensis var. hondoensis, corn, Houttuynia cordata, tomato, carrot, garlic, wild rose, malt, parsley, rye, adlay, Japanese mint, papaya, hamamelis, rose, white cedar, sunflower, loquat, coltsfoot, grapes, placenta, hazelnut, dishcloth gourd, safflower, Ficus religiosa, Paeonia suffruticosa, hop, macadamia nut, pine, marronnier, melissa, melilot, peach, malt, Rodger's bronze leaf, palm, eucalyptus, creeping saxifrage, lily, Coix lacryma-jobi var. ma-yuen, mugwort, rye, peanut, lavender, apple, litchi, lettuce, lemon, Chinese milk vetch, rosemary, camomile, agrimony, Japanese catalpa, hiba arborvitae, Euphorbia lathyris, Rabdosia japonica, Aurantii Fructus Immaturus, Senkishi, chickweed, Spirodela polyrhiza (L.) Schleid, A. capillaris Thunb., ginkgo, Chinese bellflower, chrysanthemum, Sasa albo-marginata, soapberry and weeping golden bell. Among these plant extracts, preferred are those extracts obtained from plants such as hamamelis, Paeonia suffruticosa, agrimony, Japanese catalpa, hiba arborvitae, Euphorbia lathyris, Rabdosia japonica and Aurantii Fructus Immaturus.
These plant extracts are preferably blended in the composition in an amount of from 0.0001 to 20% in terms of a dried solid content on the basis of the whole amount of the composition.

(Amino Acids)

Examples of the amino acids include neutral amino acids such as glycine, serine, cystine, alanine, threonine, cysteine, valine, phenyl alanine, mechionine, leucine, tyrosine, proline, isoleucine, tryptophan and hydroxyproline; acidic amino acids such as aspartic acid, asparagine, glutamine and glutamic acid; basic amino acids such as arginine, histidine and lysine; and betaines or amino acid derivatives such as acyl salcosines and salts thereof, acyl glutamic acids and salts thereof, acyl-β-alanines and salts thereof, glutathione, pyrrolidone carboxylic acids and salts thereof, oligo-peptides such as glutathine, carnosin, gramicidin S, tyrocidine A and tyrocidine B, and guanidine derivatives, and salts thereof, as described in JP 6-228023A.
These amino acids are preferably blended in the composition in an amount of from 0.001 to 50% on the basis of the whole amount of the composition.

(Anti-Inflammatory Agents)

Examples of the anti-inflammatory agent include glycyrrhizinic acid and salts thereof, glycyrrhethinic acid and salts thereof, ε-aminocaproic acid and salts thereof, allantoin, lysozyme chloride, guaiazulene, methyl salicylate and γ-oryzanol. Among these anti-inflammatory agents, preferred are glycyrrhethinic acid, stearyl glycyrrhethinate and ε-aminocaproic acid.
These anti-inflammatory agents are preferably blended in the composition in an amount of from 0.001 to 5% on the basis of the whole amount of the composition.

(Antioxidants)

Examples of the antioxidant include α-carotene, β-carotene, γ-carotene, ascorbic acid, tannic acid, tannins such as epicatechin, and flavonoids such as rutin. These antioxidants are preferably blended in the composition in an amount of from 0.001 to 5% on the basis of the whole amount of the composition.

(iv) Hydrophobic Compound

Examples of the hydrophobic compound used in the W/O emulsion previously produced in the present invention include those compounds that are ordinarily blended in toiletry goods for enhancing a function and an added value thereof, such as higher alcohols, sterols, silicones, fluorine-containing oil agents, and oil components.

(Higher Alcohols)

Examples of the higher alcohols include benzyl alcohol, isocetyl alcohol, isostearyl alcohol, behenyl alcohol, hexadecyl alcohol, phenylethyl alcohol, cetanol, stearyl alcohol, oleyl alcohol, 2-octyl dodecanol, batyl alcohol and 2-hexyl decanol. Among these higher alcohols, preferred are cetanol and stearyl alcohol.

(Sterols)

Examples of the sterols include cholesterol, cholesteryl isostearate, provitamin D3, campesterol, stegmastanol, stegmasterol, 5-dihydrocholesterol, α-spinasterol, palesterol, clionasterol, γ-citosterol, stegmastenol, sargasterol, apenasterol, ergostanol, citosterol, colbisterol, chondrillasterol, polyferrasterol, haliclonasterol, neospongosterol, fucosterol,

aptostanol, ergostadienol, ergosterol, 22-dihydroergosterol, brassicasterol, 24-methylenecholesterol, 5-dihydroergosterol, dehydroergosterol, fungisterol, cholestanol, coprostanol, zymosterol, 7-hetocholesterol, latosterol, 22-dehydrocholesterol, β-citosterol, cholestatrien-3β-ol, coprostanol, cholestanol, ergosterol, 7-dehydrocholesterol, 24-dehydrocholestadion-3β-ol, equilenin, equilin, estron, 17β-estradiol, androst-4-ene-3β,17β-diol, dehydroepiandrosterone, and cholesteryl alkenylsuccinates (JP 5-294989A). Among these sterols, especially preferred are cholesterol, cholesteryl isostearate and cholesteryl alkenylsuccinates.

(Silicones)

Examples of the silicones include those compounds that are ordinarily blended in toiletry products, for example, octamethyl polysiloxane, tetradecamethyl polysiloxane, methyl polysiloxane, highly-polymerized methyl polysiloxane and methylphenyl polysiloxane, and also include methyl polycyclosiloxanes such as octamethyl cyclotetrasiloxane and decamethyl cyclopentanesiloxane; trimethyl siloxysilicate; and modified silicones such as alkyl-modified silicones, polyether- and alkyl-modified silicones, amino-modified silicones, fluorine-modified silicones, alkyl-glyceryl ether-modified silicones, and modified organopolysiloxanes as described in JP 6-72851A.
Among these silicones, as the silicones blended in the detergent composition containing the W/O/W emulsion, preferred are high-viscosity silicone oils composed of modified silicones such as modified organopolysiloxanes. The kinematic viscosity of the silicone oils as measured at 25˚C is preferably 1,000 $mm^2/s$ and more preferably 7,000 $mm^2/s$ from the viewpoint of a good emulsification stability of the resultant detergent composition.
Meanwhile, the silicones blended in the W/O/W emulsion composition of the present invention preferably have a kinematic viscosity of from about 1 to about 100 $mm^2/s$ from the viewpoint of a good emulsification stability of the resultant emulsions composition.

(Fluorine-Containing Oil Agents)

The fluorine-containing oil agents are preferably perfluoro organic compounds which are kept in a liquid state at an ordinary temperature, such as perfluoropolyethers and fluorine-modified silicones. Specific examples of the perfluoro organic compounds include perfluorodecalin, perfluoroadamantane, perfluorobutyltetrahydrofuran, perfluorooctane, perfluorononane, perfluoropentane, perfluorodecane, perfluorododecane and perfluoropolyethers.

(Oil Components)

The oil components may be either volatile or non-volatile. Examples of the oil components include hydrocarbons such as solid or liquid paraffin, vaseline, crystal oil, ceresin, ozokerite, montan wax, squalane, and squalene; Eucalyptus oil, peppermint oil, camellia oil, macadamia nut oil, avocado oil, beef tallow, lard, horse grease, yolk oil, olive oil, carnauba wax, lanolin and jojoba oil; ester oils such as glycerol monostearate, glycerol distearate, glycerol monooleate, isopropyl palmitate, isopropyl stearate, butyl stearate, isopropyl myristate, neopentyl glycol dicaprylate, diethyl phthalate, myristyl lactate, diisopropyl adipate, cetyl myristate, myristyl lactate, diisopropyl adipate, cetyl myristate, cetyl lactate, 1-isostearoyl-3-myristoyl glycerol, cetyl 2-ethylhexanoate, 2-ethlhexyl palmitate, 2-octyldecyl myristate, neopentyl glycol di (2-ethylhexanoate), 2-octyldodecyl oleate, glycerol triisostearate and glyceryl di(p-methoxycinnamate)-mono(2-ethylhexanoate); higher fatty acids such as stearic acid, palmitic acid and oleic acid; natural essential oils such as rosemary, rooibos, royal jelly and hamamelis; and functional oil substances such as lignan, vitamin E, oil-soluble vitamin C, vitamin A derivatives, ceramides, structurally ceramide analogous substances, oil-soluble ultraviolet absorbers and perfumes.
In the detergent composition of the present invention, from the viewpoints of a good stability of the W/O/W emulsion in the finally obtained detergent system, among these hydrophobic compounds, preferred are silicones, and oil components such as hydrocarbons and higher fatty acids; more preferred are the hydrophobic compounds having a kinematic viscosity of 1,000 $mm^2/s$ or more; and still more preferred are high-viscosity silicone oils.

(v) Emulsifier Used in W/O Emulsion

The water (iii) optionally containing an effective substance and the hydrophobic compound (iv) are mixed together using an emulsifier having a HLB value of 7 or less to form a W/O emulsion. When using the emulsifier having a HLB value of 7 or less and exhibiting moderate hydrophilicity, the resultant W/O emulsion is kept stable.
Meanwhile, the HLB value is calculated from the following formula (1):
$$HLB = 7 + 11.7 \cdot \log(MW/MO) \quad (1)$$

wherein MW is a molecular weight of a hydrophilic moiety; and MO is a molecular weight of a lipophilic moiety.

(Emulsifier Having HLB Value of 7 or less)

Examples of the emulsifier having a HLB value of 7 or less include sorbitan fatty acid esters such as sorbitan monolaurate, sorbitan monooleate, sorbitan monoisostearate and sorbitan tristearate; glycerol fatty acid esters such as glycerol monostearate and glycerol monooleate; polyoxyethylene hardened castor oils such as POE (5) hardened castor oil, POE (7.5) hardened castor oil and POE (10) hardened castor oil; polyether-modified silicone-based surfactants such as dimethicon copolyol, cetyl dimethicon copolyol and dimethicon copolyol crosspolymers; polyoxyalkylenated glycerol fatty acid ester-based surfactants; polyglycerol fatty acid ester-based surfactants; and polyhydroxystearic acid esters of polyhydric alcohols, polyhydroxystearyl polyglycerol, crosslinked organopolysiloxane elastomers containing a polyoxyethylenated chain and/or a polyoxypropylenated chain, and isostearyl glyceryl ether.
Among these emulsifiers, preferred are polyhydroxystearic acid esters of polyhydric alcohols such as polyhydroxystearyl alkylenated glycols (for example, polyethylene glycol dipolyhydroxystearate "ARACEL P135" available from ICI Corp., and polyhydroxystearyl polyglycerol "DEHYMULUS PGPH" available from Henkel AG); polyether-modified silicone-based surfactants such as dimethicon copolyol "Silicone SC 9450" available from Shin-Etsu Chemical Co., Ltd.; and crosslinked organopolysiloxane elastomers containing a polyoxyethylenated chain and/or a polyoxypropylenated chain such as "KSG 210" available from Shin-Etsu Chemical Co., Ltd.
The amount of the emulsifier having a HLB value of 7 or less which is blended in the W/O/W emulsion of the present invention is preferably from 0.01 to 10% by mass and more preferably from 0.1 to 7% by mass from the viewpoint of a good stability of the resultant emulsion. When the amount of the emulsifier blended lies within the above specified range, the W/O/W emulsion can be stably produced.

(vi) Detergent Aqueous Solution

Examples of the detergent aqueous solution used for diluting the mixed composition (Z) prepared to obtain the detergent composition containing the W/O/W emulsion according to the present invention include aqueous solutions of alkylsulfates, alkylethersulfates, alkylbenzenesulfonates, alkyl betaines and alkyl glycosides which are ordinarily blended in toiletry products to enhance a washing performance thereof. From the viewpoints of a good washing performance required as detergents and a good stability of the resultant W/O/W emulsion composition, among these aqueous detergent solutions, especially preferred are aqueous solutions containing sodium polyoxyethylenelaurylethersulfate, laurylamide propyl betaine, etc.

[Production of W/O/W Emulsion]

The method for producing the W/O/W Emulsion of the present invention is not particularly limited, and the W/O/W Emulsion is preferably produced by a two-stage emulsification method. For example, the W/O/W Emulsion may be produced by the process including the steps of mixing the composition (X) prepared by mixing (i) 1 part by mass of the substituted polysaccharide and (ii) from 2 to 50 parts by mass of at least one water-soluble polyol, with from 1 to 90 parts by mass of the W/O emulsion (Y) previously produced by ordinary methods from (iii) from 1 to 30 parts by mass of water optionally containing an effective substance, (iv) from 0.01 to 70 parts by mass of the hydrophobic compound and (v) from 0.01 to 5 parts by mass of the emulsifier having a HLB value of 7 or less, to prepare the mixed composition (Z); and then diluting the resultant mixed composition (Z) with water.
In one preferred embodiment of the present invention, first, the substituted polysaccharide, the water-soluble polyol and water (or the substituted polysaccharide and an aqueous solution of the water-soluble polyol) are mixed with each other to prepare the composition (X), and then the thus obtained composition (X) is mixed with the W/O emulsion (Y) previously produced by ordinary methods to prepare the mixed composition (Z), and further the thus obtained mixed composition (Z) is diluted with water.
In this case, the contents of the substituted polysaccharide, the water-soluble polyol and water in the composition (X) are preferably from 1 to 10% by mass, from 10 to 90% by mass and from 10 to 90% by mass, respectively. In addition, the mass ratio of the water-soluble polyol to water (water-soluble polyol/water) is preferably from 10/90 to 90/10. In the above method, it is preferred that the water-soluble polyol and water are first mixed with each other to prepare an aqueous solution of the water-soluble polyol, and then the thus prepared aqueous solution is mixed with the substituted polysaccharide preferably while stirring to produce the composition (X).

The amount of the W/O emulsion to be mixed with the composition (X) is preferably from 0.01 to 70 parts by mass, more preferably from 0.1 to 50.0 parts by mass and still more preferably from 1.0 to 45.0 parts by mass on the basis of 1 part by mass of the substituted polysaccharide contained in the composition (X) from the viewpoint of stably forming the emulsion composition. The method of mixing the composition (X) with the W/O emulsion (Y) is not particularly limited. Under an appropriate mechanical mixing force, the W/O emulsion (Y) may be added to the composition (X) at one time or gradually continuously, or several divided parts of the given amount of the W/O emulsion (Y) may be intermittently added to the composition (X). At this time, the dropping velocity and the number of the divided parts of the W/O emulsion (Y) are not particularly limited, and may be appropriately adjusted such that a good mixed condition thereof is attained. The whole given amount of the W/O emulsion (Y) is mixed in the composition (X) to thereby obtain the mixed composition (Z).
The mass ratio of the water-soluble polyol to water (water-soluble polyol/water) in the thus obtained mixed composition (Z) is preferably from 10/90 to 99/1, more preferably from 20/80 to 95/5 and still more preferably from 30/70 to 90/10.

Also, in another preferred embodiment of the present invention, the mixed composition (Z) may be prepared in the following manner.
That is, first, the substituted polysaccharide is mixed with the W/O emulsion (Y) previously prepared by ordinary methods to prepare a composition (X'). The content of the W/O emulsion (Y) in the composition (X') is preferably from 0.01 to 70 parts by mass, more preferably from 0.1 to 50 parts by mass and still more preferably from 1.0 to 45 parts by mass on the basis of 1 part by mass of the substituted polysaccharide contained in the composition (X'). Next, the thus prepared composition (X') is mixed with an aqueous solution of the water-soluble polyol while stirring to produce the above mixed composition (Z). At this time, the content of the water-soluble polyol in the thus produced mixed composition (Z) is preferably from 2 to 50 parts by mass, more preferably from 2 to 40 parts by mass and still more preferably from 4 to 35 parts by mass on the basis of 1 part by mass of the substituted polysaccharide. The method of mixing the composition (X') with the aqueous solution of the water-soluble polyol is not particularly limited. Under an appropriate mechanical mixing force, the aqueous solution of the water-soluble polyol may be added to the composition (X') at one time or gradually continuously, or several divided parts of the given amount of the aqueous solution of the water-soluble polyol may be intermittently added to the composition (X'). At this time, the dropping velocity and the number of the divided parts of the aqueous solution of the water-soluble polyol are not particularly limited, and may be appropriately adjusted such that a good mixed condition thereof is attained. The whole given amount of the aqueous solution of the water-soluble polyol is mixed in the composition (X') to thereby obtain the mixed composition (Z).
The mass ratio of the water-soluble polyol to water (water-soluble polyol/water) in the thus obtained mixed composition (Z) is preferably from 10/90 to 99/1, more preferably from 20/80 to 95/5 and still more preferably from 30/70 to 90/10.
Next, the thus produced mixed composition (Z) is diluted with water to obtain the W/O/W emulsion of the present invention. The mixing method used upon diluting the composition with water is not particularly limited. The mixed composition (Z) and water may be mixed with each other under an appropriate mechanical force according to the viscosity of the composition (Z) and the amount of diluting water. The amount of water mixed is controlled such that the mass ratio of the mixed composition (Z) to water (mixed composition(Z)/water) is preferably from 1/99 to 99/1, more preferably from 1/99 to 65/35 and still more preferably from 1/99 to 50/50.

The emulsified particles containing the W/O emulsion (Y) as the inner phase which are present in the W/O/W emulsion of the present invention preferably have an average particle size of from 0.1 to 30 μm and more preferably from 0.3 to 20 μm. Meanwhile, the average particle size of the emulsified particles is the value determined by measuring the particle size distribution by a laser scattering method, and more specifically the value measured using a particle size distribution measuring apparatus "Model LA-910" available from HORIBA, Ltd.
Upon the measurement, 0.5 g of the emulsion is diluted with 99.5 g of physiologic saline, and the average particle size of emulsified particles in the resultant dilute solution is measured at room temperature.

The thus produced W/O/W emulsion of the present invention may be directly used as cosmetics, massage cosmetics and skin care agents, but may also contain various additives ordinarily used in these products such as surfactants, powders, dispersants, solvents, pigments, perfumes, dyes, inorganic salts, antiseptic agents and pH modifiers in order to enhance an added value of the products.

[Production of Detergent Composition Containing W/O/W Emulsion]

The method for producing the detergent composition containing the W/O/W emulsion according to the present invention is not particularly limited, and the detergent composition is preferably produced by a two-stage emulsification method. For example, the detergent composition containing the W/O/W emulsion may be produced by the process including the steps of mixing the composition (X) prepared by mixing (i) 1 part by mass of the substituted polysaccharide and (ii) from 2 to 50 parts by mass of water optionally containing at least one water-soluble polyol, with from 1 to 90 parts by mass of the W/O emulsion (Y) previously produced by ordinary methods from (iii) from 1 to 30 parts by mass of water optionally containing an effective substance, (iv) from 0.01 to 70 parts by mass of the hydrophobic compound

and (v) from 0.01 to 5 parts by mass of the emulsifier having a HLB value of 7 or less, to prepare the mixed composition (Z); and then diluting the resultant mixed composition (Z) with an aqueous detergent solution.

In this case, the contents of the substituted polysaccharide, the water-soluble polyol and water in the composition (X) are preferably from 1 to 10% by mass, from 0 to 40% by mass and from 50 to 100% by mass, respectively. In addition, the mass ratio of the water-soluble polyol to water (water-soluble polyol/water) is preferably from 0/100 to 50/50. In the above method, it is preferred that water optionally containing the water-soluble polyol is mixed with the substituted polysaccharide preferably while stirring to produce the composition (X).

The amount of the W/O emulsion (Y) to be mixed with the composition (X) is preferably from 0.01 to 70 parts by mass, more preferably from 0.1 to 50.0 parts by mass and still more preferably from 1.0 to 45.0 parts by mass on the basis of 1 part by mass of the substituted polysaccharide contained in the composition (X) from the viewpoint of stably forming the emulsion composition. The method of mixing the composition (X) with the W/O emulsion (Y) is not particularly limited. Under an appropriate mechanical mixing force, the W/O emulsion (Y) may be added to the composition (X) at one time or gradually continuously, or several divided parts of the given amount of the W/O emulsion (Y) may be intermittently added to the composition (X). At this time, the dropping velocity and the number of the divided parts of the W/O emulsion (Y) are not particularly limited, and may be appropriately adjusted such that a good mixed condition thereof is attained. The whole given amount of the W/O emulsion (Y) is mixed in the composition (X) to thereby obtain the mixed composition (Z).

Also, in a further preferred embodiment of the present invention, the mixed composition (Z) may be prepared in the following manner.

That is, first, the substituted polysaccharide is mixed with the W/O emulsion (Y) previously prepared by ordinary methods to prepare a composition (X'). The content of the W/O emulsion (Y) in the composition (X') is preferably from 0.01 to 70 parts by mass, more preferably from 0.1 to 50 parts by mass and still more preferably from 1.0 to 45 parts by mass on the basis of 1 part by mass of the substituted polysaccharide contained in the composition (X'). Next, the thus prepared composition (X') is mixed with water optionally containing the water-soluble polyol while stirring to produce the above mixed composition (Z). In this case, the content of the water-soluble polyol in the thus produced mixed composition (Z) is preferably from 0 to 50 parts by mass, more preferably from 0 to 30 parts by mass and still more preferably from 0 to 10 parts by mass on the basis of 1 part by mass of the substituted polysaccharide. The method of mixing the composition (X') with water optionally containing the water-soluble polyol is not particularly limited. Under an appropriate mechanical mixing force, water optionally containing the water-soluble polyol may be added to the composition (X') at one time or gradually continuously, or several divided parts of the given amount of water optionally containing the water-soluble polyol may be intermittently added to the composition (X'). At this time, the dropping velocity and the number of the divided parts of water optionally containing the water-soluble polyol are not particularly limited, and may be appropriately adjusted such that a good mixed condition thereof is attained. The whole given amount of water optionally containing the water-soluble polyol is mixed in the composition (X') to thereby obtain the mixed composition (Z).

Next, the thus produced mixed composition (Z) is diluted with the aqueous detergent solution to obtain the detergent composition containing the W/O/W emulsion according to the present invention. The mixing method used upon diluting the mixed composition with the aqueous detergent solution is not particularly limited. The mixed composition and the aqueous detergent solution may be mixed with each other under an appropriate mechanical force according to the viscosity of the composition (Z) and the amount of the aqueous detergent solution. The amount of the aqueous detergent solution mixed is adjusted such that the mass ratio of the mixed composition (Z) to the aqueous detergent solution (mixed composition(Z)/aqueous detergent solution) is preferably from 1/99 to 99/1, more preferably from 1/99 to 65/35 and still more preferably from 1/99 to 50/50.

The emulsified particles containing the W/O emulsion (Y) as the inner phase which are present in the detergent composition containing the W/O/W emulsion according to the present invention preferably have an average particle size of from 0.1 to 30 μm and more preferably from 0.3 to 20 μm. Meanwhile, the average particle size of the emulsified particles may be measured by the same method using the same apparatus as described above.

The thus produced detergent composition containing the W/O/W emulsion according to the present invention may be directly used, for example, as dish detergents, or toiletry products such as body shampoos, cleansing lotions and hair shampoos, but may also contain various additives ordinarily used in these products such as powders, dispersants, solvents, pigments, perfumes, dyes, inorganic salts, antiseptic agents and pH modifiers in order to enhance an added value of the products.

## EXAMPLES

The present invention is described in more detail by referring to the following Examples and Comparative Examples. Meanwhile, the term "%" means "% by mass" unless otherwise specified.

PRODUCTION EXAMPLE 1 (Production of Polysaccharide Derivative 1)

Eighty grams of potato starch available from Katayama Chemical Industries Co., Ltd., 640 g of 50% isopropyl alcohol and 5.5 g of a 48% sodium hydroxide aqueous solution were mixed with each other to prepare a slurry. The resultant slurry was stirred at room temperature for 30 min under a nitrogen atmosphere. The obtained solution was mixed with 19.0 g of a compound represented by the following formula (5):

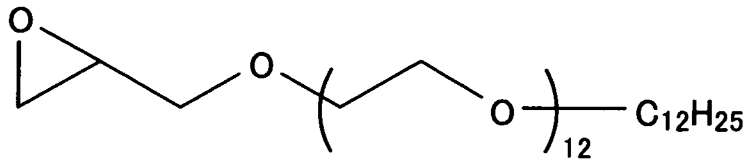

(5),

and reacted with the compound at 80°C for 8 h to effect polyoxyalkylenation thereof. After completion of the reaction, the reaction solution was neutralized with acetic acid, and the obtained reaction product was separated therefrom by filtration. The thus obtained reaction product was washed with 500 g of 50% isopropyl alcohol twice and then with 500 g of acetone twice, and then dried at 70°C under reduced pressure over a whole day and night, thereby obtaining 69.4 g of a polyoxyalkylenated starch (hereinafter referred to as a "polysaccharide derivative 1"). As a result, it was confirmed that the degree of substitution with the group (A) in the polysaccharide derivative 1 was 0.005.

PRODUCTION EXAMPLE 2 (Production of Polysaccharide Derivative 2)

To 220.0 g of the polysaccharide derivative obtained in Production Example 1, were added 200 g of 70% isopropyl alcohol, 42.6 g of sodium 3-chloro-2-hydroxypropanesulfonate and 18.0 g of a 48% sodium hydroxide aqueous solution, and the resultant mixture was subjected to sulfonation reaction at 50°C for 5h. After completion of the reaction, the reaction solution was neutralized with acetic acid, and the obtained reaction product was separated therefrom by filtration. The thus obtained reaction product was washed with 400 g of 70% isopropyl alcohol three times and then with 300 g of isopropyl alcohol twice, and then dried at 70°C under reduced pressure over a whole day and night, thereby obtaining 38.3 g of a polyoxyalkylenated and sulfonated starch (hereinafter referred to as a "polysaccharide derivative 2"). As a result, it was confirmed that the degree of substitution with 3-sulfo-2-hydroxypropyl group [group (B)] in the polysaccharide derivative 2 was 0.301.

PRODUCTION EXAMPLE 3 (Production of Polysaccharide Derivative 3)

Eighty grams of hydroxyethyl cellulose "HEC-QP100MH" available from Union Carbide Corp. (mass-average molecular weight: 1,500,000; degree of substitution with hydroxyethyl group: 1.8), 640 g of 80% isopropyl alcohol and 5.34 g of a 48% sodium hydroxide aqueous solution were mixed with each other to prepare a slurry. The resultant slurry was stirred at room temperature for 30 min under a nitrogen atmosphere. The obtained solution was mixed with 12.78 g of a compound represented by the following formula (5):

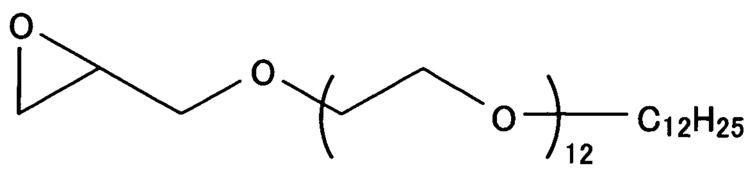

(5),

and reacted with the compound at 80°C for 8 h to effect polyoxyalkylenation thereof. After completion of the reaction, the reaction solution was neutralized with acetic acid, and the obtained reaction product was separated therefrom by filtration. The thus obtained reaction product was washed with 500 g of isopropyl alcohol twice, and then dried at 60°C under reduced pressure over a whole day and night, thereby obtaining 72.0 g of a polyoxyalkylenated hydroxyethyl cellulose (hereinafter referred to as a "polysaccharide derivative 3"). As a result, it was confirmed that the degree of

substitution with the group (A) in the polysaccharide derivative 3 was 0.004.

PRODUCTION EXAMPLE 4 (Production of Polysaccharide Derivative 4)

Eighty grams of hydroxyethyl cellulose "HEC-QP15000H" available from Union Carbide Corp. (mass-average molecular weight: 800,000; degree of substitution with hydroxyethyl group: 1.8), 640 g of 80% isopropyl alcohol and 5.34 g of a 48% sodium hydroxide aqueous solution were mixed with each other to prepare a slurry. The resultant slurry was stirred at room temperature for 30 min under a nitrogen atmosphere. The obtained solution was mixed with 13.7 g of a compound represented by the following formula (6):

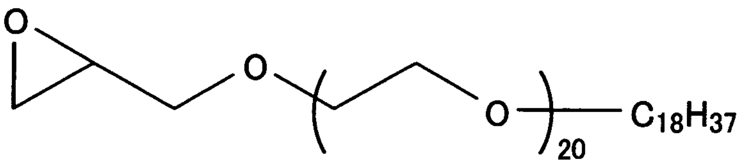

(6)

,

and reacted with the compound at 80˚C for 8 h to effect polyoxyalkylenation thereof. After completion of the reaction, the reaction solution was neutralized with acetic acid, and the obtained reaction product was separated therefrom by filtration. The thus obtained reaction product was washed with 500 g of isopropyl alcohol twice, and then dried at 60˚C under reduced pressure over a whole day and night, thereby obtaining 69.0 g of a polyoxyalkylenated hydroxyethyl cellulose (hereinafter referred to as a "polysaccharide derivative 4"). As a result, it was confirmed that the degree of substitution with the group (A) in the polysaccharide derivative 4 was 0.003.

PRODUCTION EXAMPLE 5 (Production of Polysaccharide Derivative 5)

Eighty grams of hydroxyethyl cellulose "HEC-QP4400H" available from Union Carbide Corp. (mass-average molecular weight: 500,000; degree of substitution with hydroxyethyl group: 1.8), 640 g of hydrous 80% isopropyl alcohol and 5.34 g of a 48% sodium hydroxide aqueous solution were mixed with each other to prepare a slurry. The resultant slurry was stirred at room temperature for 30 min under a nitrogen atmosphere. The obtained solution was mixed with 12.78 g of the compound represented by the above formula (5), and reacted therewith at 80˚C for 8 h to effect polyoxyalkylenation thereof. After completion of the reaction, the reaction solution was neutralized with acetic acid, and the obtained reaction product was separated therefrom by filtration. The thus obtained reaction product was washed with 500 g of isopropyl alcohol twice, and then dried at 60˚C under reduced pressure over a whole day and night, thereby obtaining 73 g of a polyoxyalkylenated hydroxyethyl cellulose (hereinafter referred to as a "polysaccharide derivative 5"). As a result, it was confirmed that the degree of substitution with the group (A) in the polysaccharide derivative 5 was 0.004.

PRODUCTION EXAMPLE 6 (Production of Polysaccharide Derivative 6)

One hundred sixty grams of hydroxyethyl cellulose "NATROZOL 250G" available from HERCULES Corp. (mass-average molecular weight 200,000; degree of substitution with hydroxyethyl group: 2.5), 1280 g of hydrous 80% isopropyl alcohol and 9.8 g of a 48% sodium hydroxide aqueous solution were mixed with each other to prepare a slurry. The resultant slurry was stirred at room temperature for 30 min under a nitrogen atmosphere. The obtained solution was mixed with 21.2 g of the compound represented by the above formula (5), and reacted therewith at 80˚C for 8 h to effect polyoxyalkylenation thereof. After completion of the reaction, the reaction solution was neutralized with acetic acid, and the obtained reaction product was separated therefrom by filtration. The thus obtained reaction product was washed with 700 g of isopropyl alcohol twice, and then dried at 60˚C under reduced pressure over a whole day and night, thereby obtaining 151 g of a polyoxyalkylenated hydroxyethyl cellulose (hereinafter referred to as a "polysaccharide derivative 6"). As a result, it was confirmed that the degree of substitution with the group (A) in the polysaccharide derivative 6 was 0.009.

PRODUCTION EXAMPLE 7 (Production of Polysaccharide Derivative 7)

One hundred sixty grams of hydroxyethyl cellulose "NATROZOL 250G" available from HERCULES Corp. (mass-average molecular weight: 200,000; degree of substitution with hydroxyethyl group: 2.5), 1280 g of hydrous 80%

isopropyl alcohol and 9.8 g of a 48% sodium hydroxide aqueous solution were mixed with each other to prepare a slurry. The resultant slurry was stirred at room temperature for 30 min under a nitrogen atmosphere. The obtained solution was mixed with 31.8 g of the compound represented by the above formula (5), and reacted therewith at 80˚C for 8 h to effect polyoxyalkylenation thereof. After completion of the reaction, the reaction solution was neutralized with acetic acid, and the obtained reaction product was separated therefrom by filtration. The thus obtained reaction product was washed with 700 g of isopropyl alcohol twice, and then dried at 60˚C under reduced pressure over a whole day and night, thereby obtaining 152 g of a polyoxyalkylenated hydroxyethyl cellulose (hereinafter referred to as a "polysaccharide derivative 7"). As a result, it was confirmed that the degree of substitution with the group (A) in the polysaccharide derivative 7 was 0.014.

PRODUCTION EXAMPLE 8 (Production of Polysaccharide Derivative 8)

One hundred sixty grams of hydroxyethyl cellulose "NATROZOL 250M" available from HERCULES Corp. (mass-average molecular weight: 500,000; degree of substitution with hydroxyethyl group: 2.5), 1280 g of hydrous 80% isopropyl alcohol and 9.8 g of a 48% sodium hydroxide aqueous solution were mixed with each other to prepare a slurry. The resultant slurry was stirred at room temperature for 30 min under a nitrogen atmosphere. The obtained solution was mixed with 47.7 g of the compound represented by the above formula (5), and reacted therewith at 80˚C for 8 h to effect polyoxyalkylenation thereof. After completion of the reaction, the reaction solution was neutralized with acetic acid, and the obtained reaction product was separated therefrom by filtration. The thus obtained reaction product was washed with 700 g of isopropyl alcohol twice, and then dried at 60˚C under reduced pressure over a whole day and night, thereby obtaining 153 g of a polyoxyalkylenated hydroxyethyl cellulose (hereinafter referred to as a "polysaccharide derivative 8"). As a result, it was confirmed that the degree of substitution with the group (A) in the polysaccharide derivative 8 was 0.021.

PRODUCTION EXAMPLE 9 (Production of Polysaccharide Derivative 9)

One hundred sixty grams of hydroxyethyl cellulose "NATROZOL 250M" available from HERCULES Corp. (mass-average molecular weight: 500,000; degree of substitution with hydroxyethyl group: 2.5), 1280 g of hydrous 80% isopropyl alcohol and 9.8 g of a 48% sodium hydroxide aqueous solution were mixed with each other to prepare a slurry. The resultant slurry was stirred at room temperature for 30 min under a nitrogen atmosphere. The obtained solution was mixed with 56.8 g of the compound represented by the above formula (5), and reacted therewith at 80˚C for 8 h to effect polyoxyalkylenation thereof. After completion of the reaction, the reaction solution was neutralized with acetic acid, and the obtained reaction product was separated therefrom by filtration. The thus obtained reaction product was washed with 700 g of isopropyl alcohol twice, and then dried at 60˚C under reduced pressure over a whole day and night, thereby obtaining 155 g of a polyoxyalkylenated hydroxyethyl cellulose (hereinafter referred to as a "polysaccharide derivative 9"). As a result, it was confirmed that the degree of substitution with the group (A) in the polysaccharide derivative 9 was 0.025.

PRODUCTION EXAMPLE 10 (Production of Polysaccharide Derivative 10)

Eighty grams of hydroxyethyl cellulose "NATROZOL 250G" available from HERCULES Corp. (mass-average molecular weight: 200,000; degree of substitution with hydroxyethyl group: 2.5), 640 g of hydrous 80% isopropyl alcohol and 4.9 g of a 48% sodium hydroxide aqueous solution were mixed with each other to prepare a slurry. The resultant slurry was stirred at room temperature for 30 min under a nitrogen atmosphere. The obtained solution was mixed with 19.02 g of the compound represented by the above formula (6), and reacted therewith at 80˚C for 8 h to effect polyoxyalkylenation thereof. After completion of the reaction, the reaction solution was neutralized with acetic acid, and the obtained reaction product was separated therefrom by filtration. The thus obtained reaction product was washed with 500 g of isopropyl alcohol twice, and then dried at 60˚C under reduced pressure over a whole day and night, thereby obtaining 74 g of a polyoxyalkylenated hydroxyethyl cellulose (hereinafter referred to as a "polysaccharide derivative 10"). As a result, it was confirmed that the degree of substitution with the group (A) in the polysaccharide derivative 10 was 0.0037.

EXAMPLES 1 TO 12

The polysaccharide derivative (i) and the polyol aqueous solution (ii) were mixed together at 60˚C at the mixing ratios shown in Tables 1 and 2, and stirred at a rate of 300 r/min to prepare a composition (X) in the form of a uniform solution. After cooling the thus obtained composition (X) to 30˚C, a W/O emulsion composition (Y) prepared by uniformly dissolving the hydrophobic compound (iv) and the emulsifier (v) using a homomixer operated at a rate of 5000 r/min

and then dropping an inner water phase component thereto while continuously mixing in the homomixer operated under the same conditions as described above, was dropped to the composition (X) at 30˚C while stirring at a rate of 300 r/min. After completion of the dropping, the resultant mixture was continuously stirred at the same rate and the same temperature as described above for 30 min or longer. Further, the obtained reaction mixture was mixed with ion-exchanged water and stirred for 30 min or longer, thereby obtaining a W/O/W emulsion composition. The thus obtained W/O/W emulsion composition was subjected to evaluation of emulsification condition immediately after the production, measurement of an average particle size of emulsified particles therein, sensory test, and evaluation of stability, by the following methods. The results are shown in Tables 1 and 2.

(1) Emulsification Condition Immediately After Production

One gram of the obtained emulsion composition was diluted with 9 g of ion-exchanged water, and an appropriate amount of the diluted emulsion composition was placed on a glass slide to observe an emulsification condition of the composition using a digital microscope "KEYENCE VH-8500" and determine the W/O/W type or O/W type of the emulsion composition from the shape of the emulsified particles contained therein.

(2) Average Particle Size of Emulsified Particles

The average particle size of the emulsified particles contained in the emulsion composition was determined as follows. That is, 0.5 g of the emulsion composition was diluted with 99.5 g of physiological saline, and the obtained diluted emulsion composition was measured at room temperature using a laser-scattering particle size distribution measuring apparatus "LA-910" available from HORIBA, Ltd.

(3) Sensory Test

The obtained emulsion composition was subjected to sensory test to evaluate touch feel thereof by three expert panelists according to the following ratings.

○: Well spread upon application onto skins with good wet feel but without stickiness and oiliness.
×: Poorly spread upon application onto skins with stickiness and oiliness.

(4) Stability

The emulsion composition was preserved at room temperature for one month. After subjecting the composition to centrifugal separation, the occurrence of leakage of pigments from the inner water phase to the outer water phase was observed by naked eyes and evaluated according to the following ratings.

○: No leakage of pigments to the outer water phase occurred.
× : Leakage of pigments to the outer water phase occurred.

TABLE 1

| | Examples | | | | | |
|---|---|---|---|---|---|---|
| | 1 | 2 | 3 | 4 | 5 | 6 |
| Emulsion composition (part) | | | | | | |
| (i) Polysaccharide derivative | | | | | | |
| Polysaccharide derivative 2 | 0.5 | | | | | |
| Polysaccharide derivative 3 | | 0.2 | | | | |
| Polysaccharide derivative 4 | | | 0.2 | | | |
| Polysaccharide derivative 5 | | | | 0.3 | | |
| Polysaccharide derivative 6 | | | | | 0.5 | 0.5 |
| Polysaccharide derivative 7 | | | | | | |

(continued)

| | Examples | | | | | |
|---|---|---|---|---|---|---|
| | 1 | 2 | 3 | 4 | 5 | 6 |
| Polysaccharide derivative 8 | | | | | | |
| Polysaccharide derivative 9 | | | | | | |
| Polysaccharide derivative 10 | | | | | | |
| (ii) Water-soluble polyol | | | | | | |
| solution | | | | | | |
| 86% Glycerol | | | | | | |
| 70% Glycerol | | | | 8 | 20 | 20 |
| 70% 1,3-Butylene glycol | | | 9 | | | |
| 30% Dipropylene glycol | | 9 | | | | |
| 50% Propylene glycol | 10 | | | | | |
| (iv) Hydrophobic compound | | | | | | |
| Vaseline | | | | 5 | | |
| Silicone oil*1 (KF96A 6cs) | | | | | | |
| Perfluoropolyether*2 | | | | | | |
| (FOMBLIN HCO4) | | | | | | |
| Sunflower oil | 3 | | | | | |
| Oleic acid | | | | | | 4 |
| Squalane | | | 2 | | | |
| Liquid paraffin | | 6 | | | 4 | |
| Olive oil | | | | | | |
| (v) Emulsifier | | | | | | |
| Crosslinked alkyl | | 1.5 | | | 1.5 | 1.5 |
| polyether-modified silicone*1 | | | | | | |
| (KSG-310) | | | | | | |
| Sorbitan monooleate | 0.5 | | | 0.5 | | |
| (SPAN80) | | | | | | |
| Isostearyl glyceryl ether | | | 0.5 | | | |
| (iii) Inner water phase | | | | | | |
| Food Yellow No. 4 | 0.001 | 0.001 | 0.001 | 0.001 | 0.001 | 0.01 |
| Phenyl alanine | 0.1 | | | | 0.1 | |
| Sodium L-glutamate | | 0.1 | | | | 0.1 |
| Sodium chloride | 0.05 | 0.05 | 0.05 | | 0.05 | |
| Magnesium sulfate | | | 1 | | | |
| Ion-exchanged water | 4 | 4 | 4 | 4 | 5 | 5 |
| Sodium chloride | 0.8 | 0.8 | 0.8 | | 0.8 | |
| Ion-exchanged water | 81.0 | 78.3 | 82.4 | 82.2 | 68.0 | 68.9 |
| Evaluation results | | | | | | |

(continued)

| | Examples | | | | | |
|---|---|---|---|---|---|---|
| | 1 | 2 | 3 | 4 | 5 | 6 |
| Emulsification condition immediately after production | W/O/W | W/O/V | W/O/W | W/O/W | W/O/W | W/O/W |
| Average particle size of emulsified particles (μm) | 2 | 12 | 15 | 3 | 18 | 19 |
| Sensory test (touch feel of emulsion) | ○ | ○ | ○ | ○ | ○ | ○ |
| Emulsification stability (after preserved at room temperature for one month) | ○ | ○ | ○ | ○ | ○ | ○ |
| Note *1: Available from Shin-Etsu Chemical Co., Ltd.<br>*2: Available from Nikko Chemicals Co., Ltd. | | | | | | |

TABLE 2

| | Examples | | | | | |
|---|---|---|---|---|---|---|
| | 7 | 8 | 9 | 10 | 11 | 12 |
| Emulsion composition (part) | | | | | | |
| (i) Polysaccharide derivative | | | | | | |
| Polysaccharide derivative 2 | | | | | | |
| Polysaccharide derivative 3 | | | | | | |
| Polysaccharide derivative 4 | | | | | | |
| Polysaccharide derivative 5 | | | | | | |
| Polysaccharide derivative 6 | | | | | | |
| Polysaccharide derivative 7 | 0.5 | 0.5 | | | | |
| Polysaccharide derivative 8 | | | 0.5 | 0.5 | | |
| Polysaccharide derivative 9 | | | | | 0.3 | |
| Polysaccharide derivative 10 | | | | | | 0.2 |
| (ii) Water-soluble polyol solution | | | | | | |
| 86% Glycerol | 20 | | | | | |
| 70% Glycerol | | 15 | | 15 | | 20 |
| 70% 1,3-Butylene glycol | | | 15 | | | |
| 30% Dipropylene glycol | | | | | 15 | |
| 50% Propylene glycol | | | | | | |
| (iv) Hydrophobic compound | | | | | | |
| Vaseline | | | | | | |
| Silicone oil*1 (KF96A 6cs) | | | 4 | | | |
| Perfluoropolyether*2 (FOMBLIN HCO4) | | | | | 1 | |
| Sunflower oil | | | | 5 | | |
| Oleic acid | | | | | | |
| Squalane | 2 | | | | | |
| Liquid paraffin | 2 | | | | 3 | 5 |

(continued)

| | Examples | | | | | |
|---|---|---|---|---|---|---|
| | 7 | 8 | 9 | 10 | 11 | 12 |
| Olive oil | | 4 | | | | |
| (v) Emulsifier | | | | | | |
| Crosslinked alkyl polyether-modified silicone*1 (KSG-310) | 1.5 | 1.5 | 1.5 | 1 | 1.5 | 0.5 |
| Sorbitan monooleate (SPAN80) | | | | | | |
| Isostearyl glyceryl ether | | | | | | |
| (iii) Inner water phase | | | | | | |
| Food Yellow No. 4 | 0.001 | 0.01 | 0.001 | 0.001 | 0.001 | 0.001 |
| Phenyl alanine | | 0.1 | | | 0.1 | 0.1 |
| Sodium L-glutamate | 0.1 | | 0.1 | 0.1 | | |
| Sodium chloride | | | | | | |
| Magnesium sulfate | | | | | | |
| Ion-exchanged water | 5 | 5 | 5 | 5 | 5 | 4 |
| Sodium chloride | | | | | | |
| Ion-exchanged water | 68.9 | 73.9 | 73.9 | 73.4 | 74.1 | 70.2 |
| Evaluation results | | | | | | |
| Emulsification condition immediately after production | W/O/W | W/O/W | W/O/W | W/O/W | W/O/W | W/O/W |
| Average particle size of emulsified particles (μm) | 15 | 16 | 17 | 15 | 20 | 16 |
| Sensory test (touch feel of emulsion) | ○ | ○ | ○ | ○ | ○ | ○ |
| Emulsification stability (after preserved at room temperature for one month) | ○ | ○ | ○ | ○ | ○ | ○ |
| Note *1: Available from Shin-Etsu Chemical Co., Ltd.<br>*2: Available from Nikko Chemicals Co., Ltd. | | | | | | |

COMPARATIVE EXAMPLES 1 TO 6

The same procedures as in Examples 1 to 12 were repeated except that polyoxyethylene hardened castor oil E.O. 60 or polyoxyethylene octyl dodecyl ether E.O. 25 as the hydrophilic emulsifier (a) was used in amounts as shown in Table 3 in place of the respective polysaccharide derivatives, thereby obtaining emulsions.
The thus obtained emulsions (which were preserved at room temperature for one month) were subjected to centrifugal separation. As a result, the leakage of pigments from the inner water phase to the outer water phase was observed in all of the emulsions. The results are shown in Table 3.
TABLE 3

| | Comparative Examples | | | | | |
|---|---|---|---|---|---|---|
| | 1 | 2 | 3 | 4 | 5 | 6 |
| Emulsion composition (part) | | | | | | |
| (a) Hydrophilic emulsifier | | | | | | |
| Polyoxyethylene hardened castor oil E.O. 60 | 0.2 | 0.2 | 1.5 | | | |
| Polyoxyethylene octyl dodecyl ether E.O. 25 | | | | 0.2 | 1.5 | 1.5 |

(continued)

| | Comparative Examples | | | | | |
|---|---|---|---|---|---|---|
| | 1 | 2 | 3 | 4 | 5 | 6 |
| (ii) Water-soluble polyol solution | | | | | | |
| 86% Glycerol | 6 | | 6 | | | |
| 70% Glycerol | | 6 | | 9 | 9 | 9 |
| (iv) Hydrophobic compound | | | | | | |
| Vaseline | | | | 5 | | |
| Silicone oil*1 (KF96A 6cs) | | | | | 5 | |
| Perfluoropolyether*2 (FOMBLIN HCO4) | 4 | | | | | |
| Sunflower oil | | | | | | 5 |
| Oleic acid | | | | | | |
| Squalane | | 4 | | | | |
| Liquid paraffin | | | 4 | | | |
| Olive oil | | | | | | |
| (v) Emulsifier | | | | | | |
| Crosslinked alkyl polyether-modified silicone*1 | | | 1.5 | | 1.5 | 1.5 |
| Sorbitan monooleate (SPAN80) | 1.5 | | | 1.5 | | |
| Isostearyl glyceryl ether | | 1 | | | | |
| (iii) Inner water phase | | | | | | |
| Food Yellow No. 4 | 0.001 | 0.001 | 0.001 | 0.001 | 0.001 | 0.001 |
| Phenyl alanine | 0.1 | | | 0.1 | 0.1 | 0.1 |
| Sodium L-glutamate | | | 0.1 | | | |
| Sodium chloride | 0.05 | 0.05 | | | | |
| Magnesium sulfate | | 1 | | | | |
| Ion-exchanged water | 4 | | | | | |
| Sodium chloride | 0.8 | 0.8 | | | | |
| Ion-exchanged water | 83.3 | 86.9 | 86.9 | 84.2 | 82.9 | 82.9 |
| Evaluation results | | | | | | |
| Emulsification condition immediately after production | ** | ** | O/W | O/W | W/O/W | W/O/W |
| Average particle size of emulsified particles (μm) | | | 15 | 11 | 16 | 17 |
| Sensory test (touch feel of emulsion) | - | - | ○ | × | ○ | ○ |
| Emulsification stability (after preserved at room temperature for one month) | - | - | × | × | × | × |
| Note *1: Available from Shin-Etsu Chemical Co., Ltd.<br>*2: Available from Nikko Chemicals Co., Ltd.<br>**: Separated | | | | | | |

EXAMPLES 13 TO 26

The polysaccharide derivative (i) and water optionally containing the polyol (ii) were mixed together at 70˚C at the mixing ratios shown in Tables 4, 5 and 6, and stirred at a rate of 300 r/min to prepare a composition (X) in the form of a uniform solution. After cooling the thus obtained composition (X) to 30˚C, a W/O emulsion composition (Y) prepared

by uniformly dissolving the hydrophobic compound (iv) and the emulsifier (v) using a homomixer operated at a rate of 10,000 r/min and then dropping an inner water phase component thereto while continuously mixing in the homomixer operated under the same conditions as described above, was dropped to the composition (X) at 30˚C while stirring at a rate of 300 r/min. After completion of the dropping, the resultant mixture was continuously stirred at the same rate and the same temperature as described above for 2 h or longer. Further, the obtained reaction mixture was mixed with ion-exchanged water and an aqueous detergent solution, and stirred for 30 min or longer, thereby obtaining a detergent composition containing a W/O/W emulsion. The thus obtained detergent composition containing the W/O/W emulsion was subjected to (1) evaluation of emulsification condition immediately after the production and (2) measurement of an average particle size of emulsified particles therein, by the above methods. In addition, the detergent composition was subjected to evaluation of emulsification stability, and sensory test as detergents, by the following methods. The results are shown in Tables 4, 5 and 6.

(5) Emulsification Stability

After subjecting the emulsion composition preserved at room temperature for 2 weeks, to centrifugal separation, the amount of pigments (for example, Food Yellow No. 5, etc.) leaked from the inner water phase to the thus separated lower layer (outer water phase) was quantitatively determined using a color difference meter "Spectrophotometer CM-2002" available from Minolta Corp., to evaluate an emulsification stability thereof (specifically, an inclusion rate of the pigments was calculated from a calibration curve separately prepared).

(6) Sensory Test

The obtained detergent composition containing the W/O/W emulsion was evaluated for touch feel thereof by three expert panelists according to the following ratings upon washing upper arms of each person therewith.

◎: Excellent feel upon use as body shampoos, and unique touch feel inherent to the W/O/W emulsion occurred after washing.
○: Unsatisfactory feel upon use as body shampoos, and only slight touch feel inherent to the W/O/W emulsion occurred after washing.
×: Poor feel upon use as body shampoos, and no unique touch feel inherent to the W/O/W emulsion occurred after washing.

TABLE 4

| | Examples | | | | |
|---|---|---|---|---|---|
| | 13 | 14 | 15 | 16 | 17 |
| Detergent composition (part) | | | | | |
| (i) Polysaccharide derivative | | | | | |
| Polysaccharide derivative 3 | | | | | |
| Polysaccharide derivative 4 | | | | | |
| Polysaccharide derivative 5 | | | | | |
| Polysaccharide derivative 6 | | | | | |
| Polysaccharide derivative 7 | 0.5 | 0.5 | 0.5 | 0.5 | 0.5 |
| Polysaccharide derivative 8 | | | | | |
| Polysaccharide derivative 9 | | | | | |
| Polysaccharide derivative 10 | | | | | |
| (ii) Water optionally containing a polyol | | | | | |
| Water | 20 | 6 | 6 | 4 | 20 |
| Glycerol | | | 4 | | |

(continued)

| | Examples | | | | |
|---|---|---|---|---|---|
| | 13 | 14 | 15 | 16 | 17 |
| 1,3-Butylene glycol | | 4 | | 6 | |
| (iv) Hydrophobic compound | | | | | |
| Silicone oil*1 (KF96A 10000cs) | 4.24 | 4.24 | 4.24 | 4.24 | 4.24 |
| (v) Emulsifier | | | | | |
| Crosslinked alkyl polyether-modified silicone (KSG-210)*1 | 0.76 | 0.76 | 0.76 | 0.76 | 0.76 |
| (iii) Inner water phase | | | | | |
| Food Yellow No. 4 | 0.002 | 0.002 | 0.002 | 0.002 | 0.002 |
| Ion-exchanged water | 2.5 | 2.5 | 2.5 | 2.5 | 2.5 |
| (vi) Aqueous detergent solution | | | | | |
| Ion-exchanged water | 57.72 | 67.72 | 67.72 | 67.72 | 54.86 |
| Sodium polyoxyethylenelaurylether sulfate (EMAL 270G)*2 | 14.28 | 14.28 | 14.28 | 14.28 | 17.14 |
| Laurylamide propyl betaine (ANHITOL 20AB)*2 | | | | | |
| 2-Ethylhexyl glyceryl ether (BENETOL GE-EH)*2 | | | | | |
| Evaluation results | | | | | |
| Emulsification condition immediately after production | W/O/W | W/O/W | W/O/W | W/O/W | W/O/W |
| Average particle size of emulsified particles (μm) | 16 | 18 | 25 | 19 | 23 |
| Emulsification stability (inclusion rate (%) of pigments after preserved at room temperature for 2 weeks) | 96 | 95 | 78 | 49 | 36 |
| Sensory test (feel upon use, touch feel after washing) | ◎ | ◎ | ◎ | ◎ | ◎ |
| Note *1: Available from Shin-Etsu Chemical Co., Ltd.<br>*2: Available from Kao Corp. | | | | | |

TABLE 5

| | Examples | | | | |
|---|---|---|---|---|---|
| | 18 | 19 | 20 | 21 | 22 |
| Detergent composition (part) | | | | | |
| (i) Polysaccharide derivative | | | | | |
| Polysaccharide derivative 3 | 0.5 | | | | |
| Polysaccharide derivative 4 | | | | | |
| Polysaccharide derivative 5 | | | | | |
| Polysaccharide derivative 6 | | | | | |
| Polysaccharide derivative 7 | | | | 0.5 | 0.5 |
| Polysaccharide derivative 8 | | | | | |
| Polysaccharide derivative 9 | | 0.5 | | | |
| Polysaccharide derivative 10 | | | 0.5 | | |
| (ii) Water optionally containing a polyol | | | | | |

(continued)

| | Examples | | | | |
|---|---|---|---|---|---|
| | 18 | 19 | 20 | 21 | 22 |
| Water | 6 | 6 | 6 | 20 | 20 |
| Glycerol | | | | | |
| 1,3-Butylene glycol | 4 | 4 | 4 | | |
| (iv) Hydrophobic compound | | | | | |
| Silicone oil*1 (KF96A 10000cs) | 4.24 | 4.24 | 4.24 | 4.24 | 4.24 |
| (v) Emulsifier | | | | | |
| Crosslinked alkyl polyether-modified silicone (KSG-210)*1 | 0.76 | 0.76 | 0.76 | 0.76 | 0.76 |
| (iii) Inner water phase | | | | | |
| Food Yellow No. 4 | 0.002 | 0.002 | 0.002 | 0.002 | 0.002 |
| Ion-exchanged water | 2.5 | 2.5 | 2.5 | 2.5 | 2.5 |
| (vi) Aqueous detergent solution | | | | | |
| Ion-exchanged water | 67.72 | 67.72 | 67.72 | 51.06 | 56.52 |
| Sodium polyoxyethylenelaurylether sulfate (EMAL 270G)*2 | 14.28 | 14.28 | 14.28 | 14.28 | 14.28 |
| Laurylamide propyl betaine (ANHITOL 20AB)*2 | | | | 6.66 | |
| 2-Ethylhexyl glyceryl ether (BENETOL GE-EH)*2 | | | | | 1.2 |
| Evaluation results | | | | | |
| Emulsification condition immediately after production | W/O/W | W/O/W | W/O/W | W/O/W | W/O/W |
| Average particle size of emulsified particles ($\mu$m) | 21 | 22 | 19 | 20 | 30 |
| Emulsification stability (inclusion rate (%) of pigments after preserved at room temperature for 2 weeks) | 94 | 93 | 89 | 21 | 20 |
| Sensory test (feel upon use, touch feel after washing) | ◎ | ◎ | ◎ | ○ | ○ |
| Note *1: Available from Shin-Etsu Chemical Co., Ltd.<br>*2: Available from Kao Corp. | | | | | |

TABLE 6

| | Examples | | | |
|---|---|---|---|---|
| | 23 | 24 | 25 | 26 |
| <u>Detergent composition (part)</u> | | | | |
| (i) Polysaccharide derivative | | | | |
| Polysaccharide derivative 3 | | | | |
| Polysaccharide derivative 4 | 0.5 | | | |
| Polysaccharide derivative 5 | | 0.5 | | |
| Polysaccharide derivative 6 | | | 0.5 | |
| Polysaccharide derivative 7 | | | | |
| Polysaccharide derivative 8 | | | | 0.5 |
| Polysaccharide derivative 9 | | | | |

(continued)

| | Examples | | | |
|---|---|---|---|---|
| | 23 | 24 | 25 | 26 |
| Polysaccharide derivative 10 | | | | |
| (ii) Water optionally containing a polyol | | | | |
| Water | 20 | 20 | 20 | 20 |
| Glycerol | | | | |
| 1,3-Butylene glycol | | | | |
| (iv) Hydrophobic compound | | | | |
| Silicone oil*1 (KF96A 10000cs) | 4.24 | 4.24 | 4.24 | 4.24 |
| (v) Emulsifier | | | | |
| Crosslinked alkyl polyether-modified silicone (KSG-210)*1 | 0.76 | 0.76 | 0.76 | 0.76 |
| (iii) Inner water phase | | | | |
| Food Yellow No. 4 | 0.002 | 0.002 | 0.002 | 0.002 |
| Ion-exchanged water | 2.5 | 2.5 | 2.5 | 2.5 |
| (vi) Aqueous detergent solution | | | | |
| Ion-exchanged water | 57.72 | 57.72 | 57.72 | 57.72 |
| Sodium polyoxyethylenelaurylether sulfate (EMAL 270G)*2 | 14.28 | 14.28 | 14.28 | 14.28 |
| Laurylamide propyl betaine (ANHITOL 20AB)*2 | | | | |
| 2-Ethylhexyl glyceryl ether (BENETOL GE-EH)*2 | | | | |
| Evaluation results | | | | |
| Emulsification condition immediately after production | W/O/W | W/O/W | W/O/W | W/O/W |
| Average particle size of emulsified particles (μm) | 18 | 16 | 21 | 25 |
| Emulsification stability (inclusion rate (%) of pigments after preserved at room temperature for 2 weeks) | 91 | 90 | 87 | 75 |
| Sensory test (feel upon use, touch feel after washing) | ◎ | ◎ | ◎ | ◎ |
| Note *1: Available from Shin-Etsu Chemical Co., Ltd.<br>*2: Available from Kao Corp. | | | | |

COMPARATIVE EXAMPLES 7 TO 12

The same procedures as in Examples 13 to 26 were repeated except that polyoxyethylene hardened castor oil E.O. 60 or polyoxyethylene octyl dodecyl ether E.O. 25 as the hydrophilic emulsifier (a) was used in amounts as shown in Table 7 in place of the respective polysaccharide derivatives, thereby attempting to obtain emulsions. However, all of the compositions obtained above failed to form a stable detergent composition containing a W/O/W emulsion. The results are shown in Table 7.

TABLE 7

| | Comparative Examples | | | | | |
|---|---|---|---|---|---|---|
| | 7 | 8 | 9 | 10 | 11 | 12 |
| Detergent composition (Dart) | | | | | | |
| (a) Hydrophilic emulsifier | | | | | | |
| Polyoxyethylene hardened | 0.5 | 0.5 | 0.5 | | | |

(continued)

| | Comparative Examples | | | | | |
|---|---|---|---|---|---|---|
| | 7 | 8 | 9 | 10 | 11 | 12 |
| castor oil E.O. 60 | | | | | | |
| Polyoxyethylene octyl dodecyl ether E.O. 25 | | | | 0.5 | 0.5 | 0.5 |
| (ii) Water optionally containing polyol | | | | | | |
| Water | 20 | 6 | 6 | 20 | 6 | 6 |
| Glycerol | | | 4 | | | 4 |
| 1,3-Butylene glycol | | 4 | | | 4 | |
| (iv) Hydrophobic compound | | | | | | |
| Silicone oil*1 (KF96A 10000cs) | 4.24 | 4.24 | 4.24 | 4.24 | 4.24 | 4.24 |
| (v) Emulsifier | | | | | | |
| Crosslinked alkyl polyether-modified silicone (KSG-210)*1 | 0.76 | 0.76 | 0.76 | 0.76 | 0.76 | 0.76 |
| (iii) Inner water phase | | | | | | |
| Food Yellow No. 4 | 0.002 | 0.002 | 0.002 | 0.002 | 0.002 | 0.002 |
| Ion-exchanged water | 2.5 | 2.5 | 2.5 | 2.5 | 2.5 | 2.5 |
| (vi) Aqueous detergent solution | | | | | | |
| Ion-exchanged water | 57.72 | 67.72 | 67.72 | 57.72 | 67.72 | 67.72 |
| Sodium polyoxyethylenelaurylether sulfate (EMAL 270G)*2 | 14.28 | 14.28 | 14.28 | 14.28 | 14.28 | 14.28 |
| Evaluation results | | | | | | |
| Emulsification condition immediately after production | ** | ** | ** | ** | ** | ** |
| Average particle size of emulsified particles (μm) | | | | | | |
| Emulsification stability (inclusion rate (%) of pigments after preserved at room temperature for 2 weeks) | 0 | 0 | 0 | 0 | 0 | 0 |
| Sensory test (feel upon use, touch feel after washing) | × | × | × | × | × | × |
| Note *1: Available from Shin-Etsu Chemical Co., Ltd.<br>*2: Available from Kao Corp.<br>**: Separated | | | | | | |

The W/O/W emulsion composition of the present invention is excellent in emulsification stability, and exhibits a good spreadability and imparts a wet feel upon use without stickiness and oiliness. Also, according to the process of the present invention, the W/O/W emulsion composition can be produced in an efficient manner.
Further, the detergent composition containing the W/O/W emulsion according to the present invention is excellent in emulsification stability, and imparts an excellent touch feel after washing without deterioration in washing performance. In addition, according to the process of the present invention, the detergent composition containing the W/O/W emulsion can be produced in an efficient manner.

INDUSTRIAL APPLICABILITY

The W/O/W emulsion composition of the present invention is excellent in emulsification stability, and exhibits a good spreadability and imparts a wet feel upon use without stickiness and oiliness, and can be applied, for example, to cosmetics, massage cosmetics and skin care agents. Also, according to the process of the present invention, the W/O/W emulsion composition as well as the detergent composition containing the W/O/W emulsion can be produced in an efficient manner.
Further, the detergent composition containing the W/O/W emulsion according to the present invention is excellent in emulsification stability, and imparts an excellent touch feel after washing without deterioration in washing performance. For example, the detergent composition is usable as a detergent having an excellent touch feel after washing without

deterioration in inherent washing performance when applied to dish detergents, or toiletry product such as body shampoos, cleansing lotions and hair shampoos.

## Claims

**1.** A W/O/W emulsion composition comprising (i) a polysaccharide derivative containing in a side chain thereof, a group represented by the following general formula (1):

$$-(OX)_n-E^1-R \qquad (1)$$

wherein X is a linear or branched divalent saturated hydrocarbon group having 1 to 6 carbon atoms; n is a number of 5 to 300; a plurality of X groups in number of n may be the same or different; $E^1$ is an ether bond (-O-) or an ester bond (-OCO- or -COO-); and R is a linear or branched hydrocarbon group having 4 to 30 carbon atoms which may be substituted with hydroxyl group.

**2.** The W/O/W emulsion composition according to claim 1, wherein the polysaccharide derivative is a substituted polysaccharide obtained by substituting a part or whole of hydrogen atoms of hydroxyl groups of a polysaccharide or a derivative thereof with at least one group selected from the group consisting of the following groups (A) to (D), or a salt thereof:

(A) a group represented by the following general formula (2):

$$-E^2-(OX)_m-E^1-R \qquad (2)$$

wherein $E^2$ is a linear or branched divalent saturated hydrocarbon group having 1 to 6 carbon atoms which may be substituted with hydroxyl group or oxo group; m is a number of 8 to 300; X, $E^1$ and R are the same as defined above; and a plurality of X groups in number of m may be the same or different;
(B) a sulfoalkyl group having 1 to 5 carbon atoms which may be substituted with hydroxyl group, or a salt thereof;
(C) a carboxyalkyl group having 2 to 6 carbon atoms which may be substituted with hydroxyl group, or a salt thereof; and
(D) a group represented by the following general formula (3):

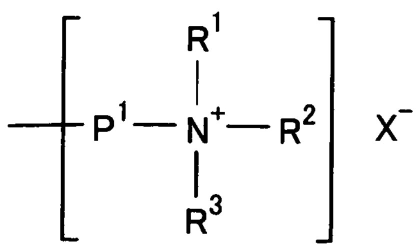
(3)

wherein $P^1$ is a linear or branched divalent saturated hydrocarbon group having 1 to 6 carbon atoms which may be substituted with hydroxyl group; $R^1$, $R^2$ and $R^3$ are respectively a linear or branched alkyl group having 1 to 3 carbon atoms which may be substituted with hydroxyl group, and may be the same or different; and $X^-$ is a hydroxyl ion, a halogen ion or an organic acid ion.

**3.** A detergent composition comprising a W/O/W emulsion containing the polysaccharide derivative as defined in claim 1.

**4.** The detergent composition according to claim 3, wherein the polysaccharide derivative contains the substituted polysaccharide as defined in claim 2.

**5.** A process for producing a W/O/W emulsion composition, comprising the steps of:

mixing a composition (X) containing (i) a polysaccharide derivative containing in a side chain thereof, a group represented by the following general formula (1):

$$-(OX)_n-E^1-R \qquad (1)$$

wherein X is a linear or branched divalent saturated hydrocarbon group having 1 to 6 carbon atoms; n is a number of 5 to 300; a plurality of X groups in number of n may be the same or different; $E^1$ is an ether bond (-O-) or an ester bond (-OCO- or -COO-); and R is a linear or branched hydrocarbon group having 4 to 30 carbon atoms which may be substituted with hydroxyl group,
and (ii) a water-soluble polyol optionally containing water, with a W/O emulsion (Y) previously produced from (iii) water, (iv) a hydrophobic compound and (v) an emulsifier having a HLB value of 7 or less, to obtain a mixed composition (Z); and
diluting the resultant mixed composition (Z) with water.

**6.** A process for producing a detergent composition containing a W/O/W emulsion, comprising the step of diluting the mixed composition (Z) as defined above, with (vi) an aqueous detergent solution.

**7.** The process according to claim 5 or 6, wherein the water (iii) contains an effective substance.

**8.** The process according to claim 6 or 7, wherein the hydrophobic compound (iv) is a compound having a kinematic viscosity of 1,000 $mm^2/s$ or more.

**9.** The process according to any one of claims 5 to 8, wherein the polysaccharide derivative is a substituted polysaccharide obtained by substituting a part or whole of hydrogen atoms of hydroxyl groups of a polysaccharide or a derivative thereof with at least one group selected from the group consisting of the following groups (A) to (D), or a salt thereof:

(A) a group represented by the following general formula (2):

$$-E^2-(OX)_m-E^1-R \qquad (2)$$

wherein $E^2$ is a linear or branched divalent saturated hydrocarbon group having 1 to 6 carbon atoms which may be substituted with hydroxyl group or oxo group; m is a number of 8 to 300; X, $E^1$ and R are the same as defined above; and a plurality of X groups in number of m may be the same or different;
(B) a sulfoalkyl group having 1 to 5 carbon atoms which may be substituted with hydroxyl group, or a salt thereof;
(C) a carboxyalkyl group having 2 to 6 carbon atoms which may be substituted with hydroxyl group, or a salt thereof; and
(D) a group represented by the following general formula (3):

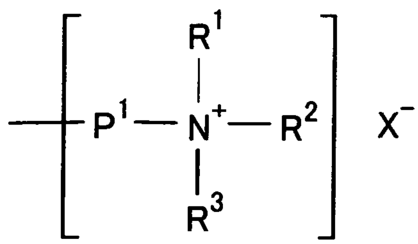

(3)

wherein $P^1$ is a linear or branched divalent saturated hydrocarbon group having 1 to 6 carbon atoms which may be substituted with hydroxyl group; $R^1$, $R^2$ and $R^3$ are respectively a linear or branched alkyl group having 1 to 3 carbon atoms which may be substituted with hydroxyl group, and may be the same or different; and $X^-$ is a hydroxyl ion, a halogen ion or an organic acid ion.

**10.** The process according to any one of claims 5, 7 and 9, comprising the steps of:

mixing the composition (X) containing 1 part by mass of the polysaccharide derivative (i) and 2 to 50 parts by mass of the water-soluble polyol (ii), with the W/O emulsion (Y) containing 1 to 30 parts by mass of the water (iii), 0.01 to 70 parts by mass of the hydrophobic compound (iv) and 0.01 to 5 parts by mass of the emulsifier (v) having a HLB value of 7 or less, to obtain the mixed composition (Z); and

diluting the resultant mixed composition (Z) with water.

**11.** The process according to claim 10, wherein a mass ratio of the water-soluble polyol to water (water-soluble polyol/ water) in the mixed composition (Z) is from 10/90 to 99/1.

**12.** The process according to any one of claims 6, 8 and 9, comprising the steps of:

preparing the composition (X) containing 1 part by mass of the polysaccharide derivative (i) and 2 to 50 parts by mass of the water-soluble polyol (ii) optionally containing water, and the W/O emulsion (Y) containing 1 to 30 parts by mass of the water (iii), 0.01 to 70 parts by mass of the hydrophobic compound (iv) and 0.01 to 5 parts by mass of the emulsifier (v) having a HLB value of 7 or less;
mixing the composition (X) with the W/O emulsion (Y) to prepare the mixed composition (Z); and
diluting the resultant mixed composition (Z) with the aqueous detergent solution (vi).

**13.** A cosmetic comprising the W/O/W emulsion composition as defined in claim 1 or 2.

**14.** A massage cosmetic comprising the W/O/W emulsion composition as defined in claim 1 or 2.

**15.** A skin care agent comprising the W/O/W emulsion composition as defined in claim 1 or 2.

**16.** A dish detergent comprising the detergent composition containing the W/O/W emulsion as defined in claim 3 or 4.

**17.** A toiletry product comprising the detergent composition containing the W/O/W emulsion as defined in claim 3 or 4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002275029 A
- JP 2001025360 A
- WO 0073351 A
- JP 2003226612 A
- JP 6228023 A
- JP 5294989 A
- JP 6072851 A